# EUROPEAN PATENT APPLICATION

(11) **EP 1 547 552 A1**
(43) Date of publication of application: **29.06.2005**
(21) Application number: 03784573.2
(22) Date of filing: 07.08.2003
(51) Int. Cl.: A61F 13/15

(54) **ABSORPTIVE ARTICLE**

(30) Priority: 08.08.2002 JP 2002230910; 26.09.2002 JP 2002281389
(71) Applicant: Daio Paper Corporation, Shikokuchuo-shi, Ehime 799-0492 (JP)
(72) Inventor: HANAO, Hiroyuki, DAIO PAPER CONVERTING. CO., LTD., Iyomishima-shi, Ehime 799-0431 (JP); KONDO, Koji, c/o DAIO PAPER CONVERTING. CO., LTD., Iyomishima-shi, Ehime 799-0431 (JP); TORIGOSHI, Keiji, DAIO PAPER CONVERTING. CO., LTD., Iyomishima-shi, Ehime 799-0431 (JP)
(74) Representative: Dealtry, Brian
(86) International application number: PCT/JP2003/010062
(87) International publication number: WO 2004/014272

(57) **Abstract**

An absorbent article which can be thin without decreasing absorption capability and strength. An absorbent body having a pulp fiber and a super absorbent polymer, in which a content of the super absorbent polymer is not less than 55% by weight, is provided. The absorbent body may be covered with a top sheet which is liquid permeable and uses a non-woven fabric.

## Description

### Field of the Invention

The present invention relates to an absorbent article which is used as an absorbent core of a disposable diaper, a sanitary product or the like.

### Background Art

An earlier developed absorbent article is formed by covering an absorbent body comprising a pulp and super absorbent polymers with a liquid permeable upper sheet, and the content of the super absorbent polymers to the pulp does not exceed 50% by weight (for example, refer to the Patent Document 1 described below). In the absorbent article having such structure, since enough strength of the absorbent body can be retained by tangles of fibers of the pulp, a top sheet and the absorbent body are adhered by a hot-melt adhesive, a polyvinyl alcohol, or a heat-welding fiber.

### Patent Document 1: Tokuhyo-2002-512082

The above described absorbent article has a trend to be made thin, and there is a demand to make the absorbent article itself thin. However, when the absorbent article itself becomes thin, the amount of super absorbent polymers in the absorbent article necessarily decreases, thereby causing a problem to decrease absorption capacity.

For the above described reasons, efforts have been made to make the absorbent body thin without reducing the content of the super absorbent polymers contained in the absorbent body. In this case, although the content of the pulp contained in the absorbent body needs to be reduced, when the content of the pulp is less than 50% by weight, another problem that retaining the strength of the absorbent body becomes difficult occurs because the super absorbent polymers itself do not have strength retention capability. In this case, the method to adhere the front surface sheet and the absorbent body by a hot-melt adhesive, a polyvinyl alcohol, or a heat seal fiber similar to the above described earlier technique is not enough.

Also, earlier, the absorbent article such as a disposable diaper, sanitary napkin or the like generally comprises a permeable upper sheet covering the front surface side which a body of a wearer contacts, an impermeable lower sheet covering the rear surface side thereof, and an absorbent body interposed between these sheets. There is a demand for such absorbent articles from general users to be excellent in absorptivity and wearability, and to be able to purchase at a low cost.

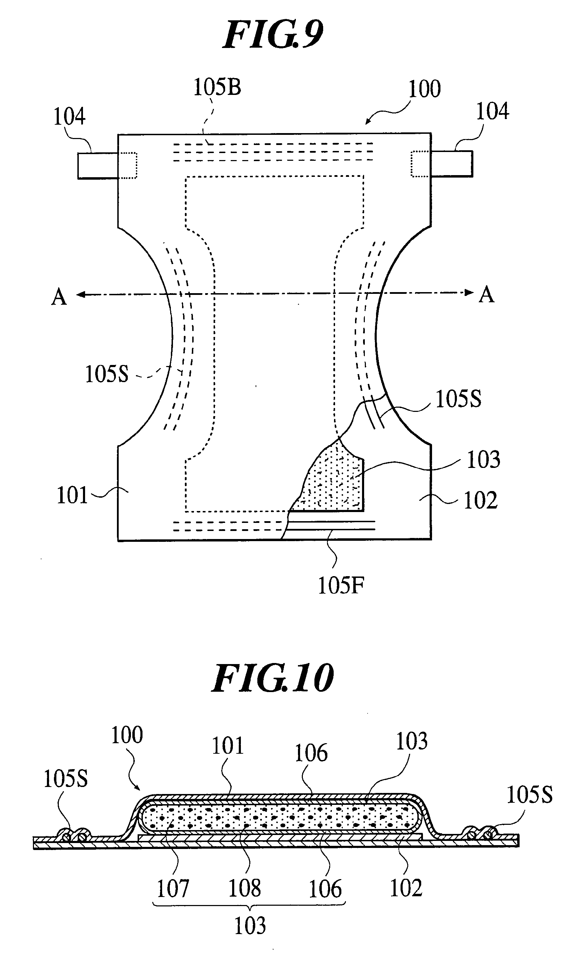

FIG. 9 is a development plan view of a taped disposable diaper 100 as an example of an earlier developed absorbent article as seen from the front surface side, and FIG. 10 is a sectional view along the line A-A in FIG. 9. As shown in the figures, the disposable diaper 100 is one in which a disposable diaper body itself formed by an absorbent body 103 interposed between a permeable upper sheet 101 and an impermeable lower sheet 102 is formed into an approximately sandglass shape. The disposable diaper 100 is provided with fastening tapes 104 and an elastic and flexible member 105B at the back portion side for holding the disposable diaper body to fit a body, and similarly provided with an elastic and flexible member 105F at the abdominal portion side, and elastic and flexible members 105S at the leg opening portion.

The above absorbent body 103 is, as shown in FIG. 10, formed by covering a mixed layer of pulp fibers 107 and super absorbent polymers 108 with crepe papers 106. Such structured disposable diaper 100 temporarily holds human liquid such as urine or the like penetrated through the permeable upper sheet 101 at the pulp fibers 107, and thereafter holds the human liquid at the super absorbent polymers 108. At this time, the super absorbent polymers 108 swell, and the volume of the absorbent body 103 increases. Accordingly, increasing the amount of the super absorbent polymers 108 has disadvantages that the amount of the pulp fibers 107 in the absorbent body 103 relatively decreases, thereby reducing the absorption speed and tangling of the fibers, which results in deteriorating shape retaining property of the absorbent body 103.

On the contrary, to improve this shape retaining property, when the amount of the super absorbent polymers 108 is reduced and the amount of the pulp fibers 107 is relatively increased to form the disposable diaper 100, there are disadvantages such as decreasing motor function at leg opening portions and deteriorating the appearance because a crotch portion becomes bulky when wearing it, increasing the production cost, or the like. Therefore, in view of such absorbability, wearability, cost or the like, generally, an earlier developed absorbent article has been made to have a total thickness of about 5-10mm.

However, an ideal absorbent article which general users desire, that is, one which is excellent in absorbability and wearability and can be purchased at a low price is far from achievement, and recently, there is a strong demand of an absorbent article which has a thickness approximately equal to the sheet, and is lightweight and compact.

The present invention has been developed in view of the above points, and an object of the present invention is to make an absorbent article thin without decreasing absorption capability.

### Disclosure of the Invention

To solve the above problems,' in accordance with a first aspect of the present invention, an absorbent article comprises:
an absorbent body comprising a pulp fiber and a super absorbent polymer, in which a content of the super absorbent polymer is not less than 55% by weight.
According to such structure, absorption capability can be improved.

Preferably, this absorbent article further comprises a top sheet which is liquid permeable and uses a non-woven fabric to cover the absorbent body.

Accordingly, when the content of the super absorbent polymers in the absorbent body is not less than 55% by weight, the content of the pulp relatively decreases, which makes it difficult to retain the strength of the absorbent article because the super absorbent polymers itself do not have strength retaining capability. However, the use of the non-woven fabric as the top sheet allows the absorbent article to retain the strength. Thus, the absorbent article can be thin without decreasing absorption capability and the strength. Also, since the content of the super absorbent polymers increases, the yield may decline due to spill of the super absorbent polymers. However, the use of the non-woven fabric as the top sheet can prevent spill of the super absorbent polymers, thereby improving the yield.

Preferably, in this absorbent article, a fiber thickness, a wet strength, and a basis weight of the non-woven fabric in the top sheet are set to be not more than 2.0 denier, not less than 300g/25mm, and not less than 10g/m², respectively.

Accordingly, the strength of the non-woven fabric used as the top sheet increases, so that the absorbent article can retain sufficient strength.

Moreover, at least a portion of the non-woven fabric in the top sheet may be subjected to a hydrophilic treatment. Accordingly, liquid permeation from the non-woven fabric can equally occur, so that absorption capability can be further improved and reflux of liquid can be prevented.

Moreover, preferably, in the above described absorbent article, the super absorbent polymer further meets the following 1) to 3) absorption capability conditions:
1) an absorption speed of 30cc of artificial urine is 50 seconds or less,
2) an absorbed amount of artificial urine under a pressure of 20g/cm² is 28cc/g or more, and
3) a moisture absorbing blocking rate is 50% or less.

The use of such super absorbent polymers is successful in providing the absorbent article which is excellent in absorbability and wearability, and can be produced at a low cost. Especially, thickness of the absorbent article can be thin, so that the absorbent article which is lightweight and compact as a whole can be obtained, thereby achieving the remarkable effect of giving comfortable fitting and free motor function to the wearer, and enabling to reduce the production cost.

Moreover, the absorbent body may have a structure comprising a single layer of the pulp fiber and a mixed layer of the pulp fiber and the super absorbent polymer. Such structure can provide the absorbent article which is excellent in absorptivity and wearability, and can be produced at a low cost.

### Brief Description of the Drawings

FIG. 1 is a longitudinal sectional view of a disposable diaper in which an absorbent article according to the first embodiment of the present invention is used as an absorbent core;
FIG. 2 is a longitudinal sectional view of the absorbent article according to the first embodiment of the present invention;
FIG. 3 is a longitudinal sectional view showing a modified example of the absorbent article of the present invention;
FIG. 4 is a development plan view of a disposable diaper according to the second embodiment of the present invention when seen from the front surface side;
FIG. 5 is a sectional view along the line B-B in FIG. 4;
FIG. 6 is an expanded view of a main portion (layered structure of the absorbent body) in FIG. 5;
FIGS. 7A to 7C are explanation views of analytical methods of absorption capability of super absorbent polymers of the present invention, wherein FIG. 7A is an explanation view of an analytical method of absorption speed, FIG. 7B is an explanation view of an analytical method of absorbed amount, and FIG. 7C is an explanation view of an analytical method of moisture absorbing blocking rate;
FIGS. 8A and 8B are longitudinal sectional views showing another structural example of the absorbent body according to the present invention, respectively;
FIG. 9 is a development plan view of an earlier developed disposable diaper when seen from the front surface side; and
FIG. 10 is a sectional view along the line A-A in FIG. 9.

### Best Mode for Carrying Out the Invention

Hereinafter, similar to the above description, the content of the present invention will be described in detail based on the embodiment in a case that an absorbent article is a disposable diaper. The present invention is not necessarily limited to the embodiments below, and any change of the structure may be made without departing from the scope of the claims.

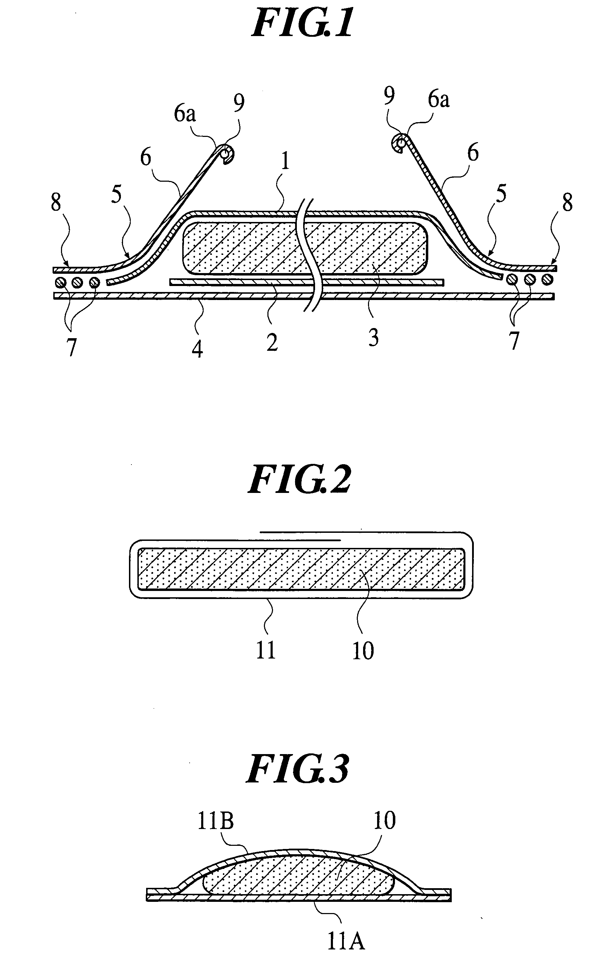

FIG. 1 is a longitudinal sectional view of a disposable diaper in which an absorbent article according to the first embodiment of the present invention is used as an absorbent core. This disposable diaper comprises a liquid permeable upper sheet 1, a liquid impermeable lower sheet 2, and a liquid retaining absorbent core 3 interposed between the liquid permeable upper sheet 1 and the liquid impermeable lower sheet 2. An outer layer non-woven fabric 4 is arranged at the lower side of the liquid impermeable lower sheet 2, and water-repellent non-woven fabrics 6, 6 for forming three-dimensional gathers 5, 5 are arranged on the liquid permeable upper sheet 1 at both right and left side edge portions of the liquid retaining absorbent core 3. The both side edge portions of the external layer non-woven fabric 4 and the bases of the water-repellent non-woven fabrics 6, 6 are both extend outward from the right and left both side edge portions of the liquid retaining absorbent core 3, and leg elastic members 7, 7 are held and sandwiched at the extending portions to form a pair of right and left leg gathers 8, 8. A free edge portion 6a of each water-repellent non-woven fabric 6 is provided with a three-dimensional gather elastic member 9 for forming the three-dimensional gather.

As shown in FIG. 2, the liquid retaining absorbent core 3 is formed by covering an absorbent body 10 in which the content of super absorbent polymers to pulp is not less than 55% by weight with a top sheet 11, and a non-woven fabric is used as the top sheet 11. As shown in FIG. 3, there may be a structure to form the liquid retaining absorbent core 3 by sandwiching the absorbent body 10 between two top sheets 11A, 11B which are adhered at peripheries.

For example, the above super absorbent polymers to be applied include various kinds of ones which have been known such as sodium polyacrylate (acrylic acid - vinyl alcohol) copolymer, cross-linking sodium polyacrylate, (starch - acrylic acid) graft copolymer, (isobutylene - maleic anhydride) copolymer and saponified material thereof, polyaspartic acid, or the like. One of them may be used independently, or a plurality of the polymers may be mixed at an appropriate rate.

The fibers forming the non-woven fabric used in the above top sheet 11 is preferably SMS. The thickness of the fiber, the wet strength, and the basis weight of the non-woven fabric are not more than 2.0 denier, not less than 300g/mm, and not less than 10g/m², respectively. When the thickness of the non-woven fabric is more than 2.0 denier, the non-woven fabric itself becomes non-dense, which would cause the yield of the super absorbent polymers to decline. When the wet strength of the non-woven fabric is less than 300g/25mm, sufficient strength cannot be obtained with the content of the super absorbent polymers not less than 55% by weight, which may result in causing twist or crack of the absorbent body 10 in use. When the basis weight of the non-woven fabric is less than 10g/m², the non-woven fabric itself becomes non-dense, which would cause the yield of the super absorbent polymers to decline.

Further, the non-woven fabric used in the above top sheet 11 is subjected to hydrophilic treatment. The hydrophilic treatment is preferably performed to both surfaces of the non-woven fabric, however, it may be performed to only one surface or may be partially performed.

According to the above structure, when the content of the super absorbent polymers is not less than 55% by weight, the content of the pulp relatively decreases, which makes it difficult to retain the strength of the absorbent body 10 because the super absorbent polymers itself do not have strength retaining capability. However, the use of the non-woven fabric as the top sheet 11 allows the absorbent body 10 to retain the strength. Thus, the absorbent article can be thin without decreasing absorption capability and the strength.

Also, the thickness of the fiber, the wet strength, and the basis weight of the non-woven fabric used in the top sheet 11 are set to be not more than 2.0 denier, not less than 300g/mm, and not less than 10g/m², respectively, so that the absorbent article can retain sufficient strength.

Since the non-woven fabric is subjected to the hydrophilic treatment, the liquid permeation from the non-woven fabric can evenly occur, so that absorption capability can be further improved and reflux of liquid can be prevented.

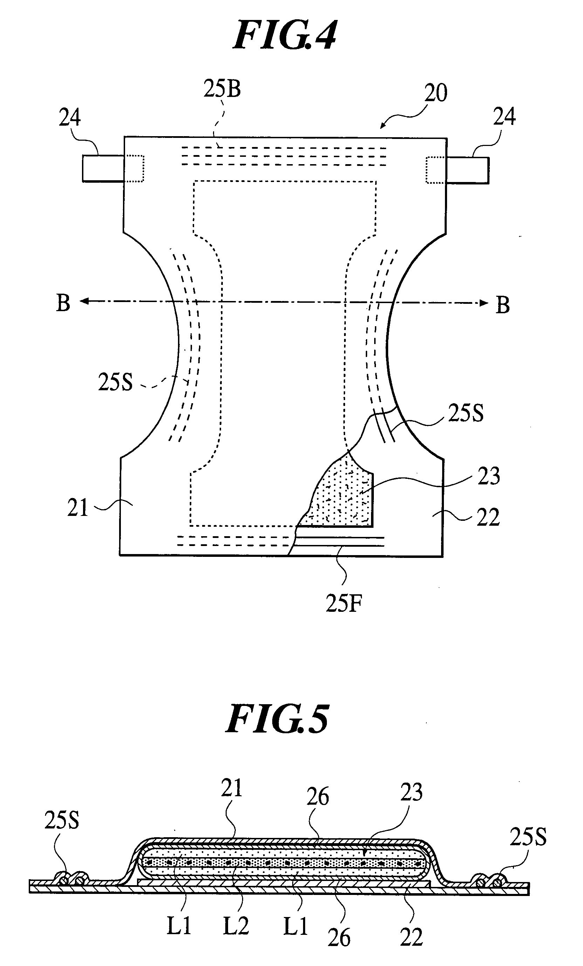

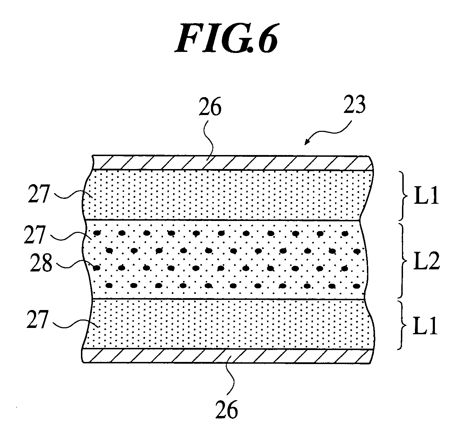

FIG. 4 is a development plan view of a disposable diaper 20 according to the second embodiment of the present invention when seen from the front surface side, FIG. 5 is a sectional view along the line B-B in FIG. 4, and FIG. 6 is an expanded view of a main portion in FIG. 5. As shown in figures, the disposable diaper 20 has a structure substantially the same except the internal structure of an absorbent body 23 compared to the earlier developed disposable diaper 100. Thus, the components corresponding to those of the disposable diaper 100 have corresponding numbers and characters of twenties.

That is, in the disposable diaper 20, the disposable diaper body is formed by the absorbent body 23 interposed between a liquid permeable upper sheet 21 and a liquid impermeable lower sheet 22. The disposable diaper 20 is provided with fastening tapes 24 and an elastic and flexible member 25B comprising a rubber string or the like for holding and the disposable diaper body to fit to a body at the back portion side which is formed into an approximately sandglass shape as a whole, and similarly provided with an elastic and flexible member 25F at the abdominal portion side and elastic and flexible members 25S at the leg opening portions.

A material which is soft and has a pleasant texture with liquid permeability such as a woven fabric, a non-woven fabric, a porous sheet or the like is used for the liquid permeable upper sheet 21. A material which is soft and has a pleasant texture with liquid impermeability such as a polyethylene or a waterproof film , a composite thereof, or a composite of a film, a woven fabric and the like is used for the liquid impermeable lower sheet 22.

The absorbent body 23 is, as shown in the sectional views of FIGS. 5 and 6, formed by covering three layers of a single layer L1 of pulp fibers 27, a mixed layer L2 of the pulp fibers 27 and the super absorbent polymers 28, and the single layer L1 of the pulp fibers 27 from the front surface side with a crepe paper 26. The thicknesses of the single layer L1 of the pulp fibers 27 and the mixed layer L2 of the pulp fibers 27 and the super absorbent polymers 28 are set to be L1=0-4mm, and L2=1-8mm, respectively, so that the thickness of the entire absorbent body 23 is set to be 1-10mm, which is much thinner than that of the above described earlier developed disposable diaper 100.

As a material for the pulp fibers 27, flocculent pulp or the like which is publicly known is applied, and poly (sodium acrylate) or acrylic acid - vinyl alcohol copolymer or the like is preferably used as the super absorbent polymers 28.

The absorbent body 23 is designed such that, in the three layers, the whole content of the super absorbent polymers 28 is 55% by weight, the whole content of the pulp fibers 27 is 45% by weight, and further the super absorbent polymers 28 meet the following 1) to 3) absorption capability conditions:
1) an absorption speed of 30cc of artificial urine is 50 seconds or less,
2) an absorbed amount of artificial urine under a pressure of 20g/cm² is 28cc/g or more, and
3) a moisture absorbing blocking rate (gel strength) is 50% or less.

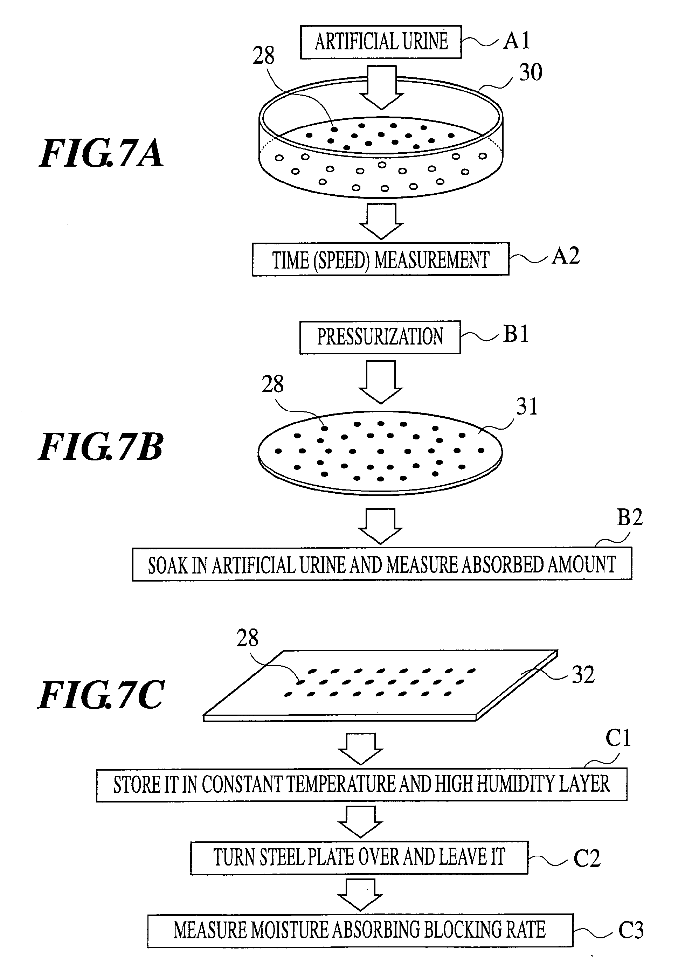

The above absorption capability of the super absorbent polymers 28 is measured by the analytical methods shown in FIGS. 7A to 7C. FIG. 7A to 7C are explanation views typically showing the methods of analyzing the absorption speed, the absorbed amount under a pressure, and the moisture absorbing blocking rate, respectively.

First, regarding the absorption speed of the super absorbent polymers 28, as shown in FIG. 7A, 1g of the super absorbent polymers 28 are dispersed in the Schale 30 having a diameter of 90mm, putting 30cc of artificial urine therein (A1), and the time (second) for the artificial urine to be absorbed in the super absorbent polymers 28 is measured. As a result of eager experiments by the inventor and the like, it has found out that the absorption speed is preferably not more than 50 seconds in terms of absorption capability of the super absorbent polymers 28. When the absorption speed exceeds 50 seconds, hydrophilic property of the super absorbent polymers 28 becomes high, therefore, exposing the wet back of urine and easily getting a diaper rash.

Next, regarding the absorbed amount of the super absorbent polymers 28, as shown in FIG. 7B, 0.2g of the super absorbent polymers 28 are dispersed in an area of 40mm diameter on a filter paper 31 having a diameter of 50mm under the pressure of 20g/cm² (B1), and the absorbed amount (cc/g) is measured by soaking it in artificial urine (B2). Similar to the above, as a result of the experiment, it has found out that the absorbed amount of the super absorbent polymers 28 is preferably not less than 28cc/g in terms of absorption capability. When the absorbed amount of the super absorbent polymers 28 is less than 28cc/g, similar to the case of the absorption speed, the wet back of urine exposes and a wearer easily gets a diaper rash.

Regarding the moisture absorbing blocking rate of the super absorbent polymers 28, as shown in FIG. 7C, 2g of the super absorbent polymers 28 are dispersed in an area of 100mm×10mm on a steel plate 32 of 150mm×150mm (square), storing the steel plate 32 in the constant temperature and high humidity bath in which the temperature is 40°C and the humidity is 90% for 30 seconds (C1), turning the steel plate 32 over and leaving it for one minute (C2), and the moisture absorbing blocking rate of the super absorbent polymers 28 is measured based on the calculating formula, that is, super absorbent polymers remaining in the steel plate 32 / total weight × 100 = moisture absorbing blocking rate (%) (C3). Similar to the above, as a result of the experiment, it has found out that the moisture absorbing blocking rate of the super absorbent polymers 28 is preferably not more than 50%. In a case that the moisture absorbing blocking rate of the super absorbent polymers 28 is more than 50%, when conveying the super absorbent polymers 28 to the disposable diaper producing machine, the polymers adhere with each other in the atmosphere of normal humidity, thereby deteriorating dispersibility, thus, failing to equally disperse the super absorbent polymers 28. Also, polymer particles adhere in the carrier tube, the disposable diaper producing equipment or the like to form rust, so that overhaul is often performed. Thus, the producing equipment needs to be stopped every time the overhaul is performed, so that it has found out that the cost increases.

According to the disposable diaper 20 which was made by using the absorbent body 23 having the structure as described above, the pulp fibers 27 and the super absorbent polymers 28 are properly included, so that human liquid such as urine is promptly absorbed in the absorbent body 23. Moreover, the total thickness can be thin, not more than 5mm. Thus, the disposable diaper 20 which is lightweight and compact can be produced at a low cost.

In the above embodiments, the absorbent body 23 is formed to have the three layers, however, it is not limited thereto in the present invention, and may be changed as shown in FIGS, 8A and 8B on condition that the contents of the super absorbent polymers and the pulp fibers are not less than 55% by weight and not more than 45% by weight, respectively.

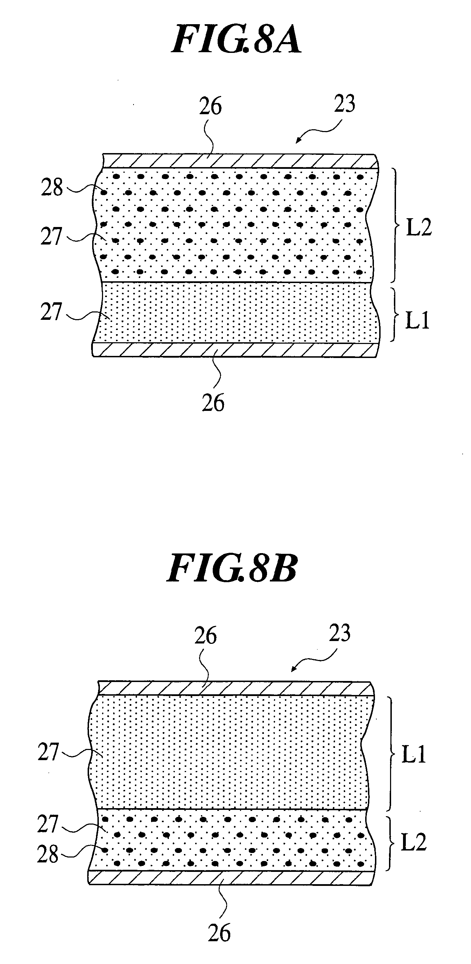

That is, the absorbent body 23 shown in FIG. 8A is formed such that two layers of the mixed layer L2 of the pulp fibers 27 and the super absorbent polymers 28 on the front surface side, and the single layer L1 of the pulp fibers 27 which is the lower layer of the mixed layer L2 are covered with the crepe paper 26. The thickness of the mixed layer L2 of the pulp fibers 27 and the super absorbent polymers 28 is set to be L2=1-6mm, and the thickness of the single layer L1 of the pulp fibers 27 is set to be L1=0-4mm.

Similarly, the absorbent body 23 shown in FIG. 8B is formed such that two layers of the single layer L1 of the pulp fibers 27 on the front surface side and the mixed layer L2 of the pulp fibers 27 and the super absorbent polymers 28 which is the lower layer of the single layer L1 are covered with the crepe paper 16. The thickness of the single layer L1 of the pulp fibers 27 is set to be L1=0-4mm, and the thickness of the mixed layer L2 of the pulp fibers 27 and the super absorbent polymers 28 is set to be L2=1-6mm.

It has found out that the absorbent body 23 having two layer structure also can obtain the effect same as the above embodiments when the super absorbent polymers 28 meet the above described 1) to 3) absorption capability conditions.

### Industrial Applicability

As described above, the content of the present invention was explained about the disposable diaper, however, it is to be understood that the present invention may be applied to publicly known absorbent articles such as a sanitary napkin, a urine absorption pad or the like.

## Claims

1. An absorbent article comprising:
an absorbent body comprising a pulp fiber and a super absorbent polymer, in which a content of the super absorbent polymer is not less than 55% by weight.

2. The absorbent article as claimed in claim 1, further comprising a top sheet which is liquid permeable and uses a non-woven fabric to cover the absorbent body.

3. The absorbent article as claimed in claim 2, wherein a fiber thickness, a wet strength, and a basis weight of the non-woven fabric in the top sheet are set to be not more than 2.0 denier, not less than 300g/25mm, and not less than 10g/m², respectively.

4. The absorbent article as claimed in any one of claims 1 to 3, wherein at least a portion of the non-woven fabric in the top sheet is subjected to a hydrophilic treatment.

5. The absorbent article as claimed in claim 1, wherein the super absorbent polymer further meets the following 1) to 3) absorption capability conditions:
1) an absorption speed of 30cc of artificial urine is 50 seconds or less,
2) an absorbed amount of artificial urine under a pressure of 20g/cm² is 28cc/g or more, and
3) a moisture absorbing blocking rate is 50% or less.

6. The absorbent article as claimed in claim 5, wherein the absorbent body comprises a single layer of the pulp fiber and a mixed layer of the pulp fiber and the super absorbent polymer.
